# EUROPEAN PATENT SPECIFICATION

(11) **EP 1 383 579 B1**
(45) Date of publication and mention of the grant of the patent: **23.03.2005**
(21) Application number: 01961133.4
(22) Date of filing: 03.08.2001
(51) Int. Cl.: A61P 25/00, A61K 31/4412, A61K 31/205

(54) **COMPOSITION, CONTAINING CARNITINE AND HUPERZIN FOR THE PREVENTION OR TREATMENT OF LEARNING DISORDERS IN CHILDREN SUFFERING FROM ATTENTION DEFICIT/HYPERACTIVE DISORDER**
CARNITIN UND HUPERZIN ENTHALTENDE ZUSAMMENSETZUNGEN ZUR BEHANDLUNG UND VORBEUGUNG VON HYPERAKTIVITÄT MIT AUFMERKSAMKEITSTÖRUNGEN BEI KINDERN
COMPOSITION POUR LA PREVENTION OU LE TRAITEMENT DE TROUBLES D'APPRENTISSAGE CHEZ LES ENFANTS SOUFFRANT DE TROUBLE D'HYPERACTIVITE AVEC DEFICIT DE L'ATTENTION

(30) Priority: 23.04.2001 IT RM20010218
(43) Date of publication of application: 28.01.2004
(73) Proprietor: SIGMA-TAU Industrie Farmaceutiche Riunite S.p.A., 00144 Roma (IT)
(72) Inventor: GAETANI, Franco, I-00040 Ariccia (IT)
(74) Representative: Cavattoni, Fabio
(86) International application number: PCT/IT2001/000430
(87) International publication number: WO 2002/085341

(56) References cited:
- WO-A-98/48785
- WO-A-99/04785
- MOECK: "Selaginis Herba" 1998 , SPRINGER , BERLIN HEIDELBERG XP002222987 3 page 129, right-hand column -page 132, left-hand column, paragraph 1

## Description

The present invention relates to a composition for the prevention and treatment of learning disorders in children suffering from Attention Deficit/Hyperactive Disorder (ADHD).

The criteria for the diagnosis of ADHD are precisely set forth in the Diagnostic and Statistical Manual of Mental Disorders, Fourth Edition (DSM-IV), pp. 82-85, published by the American Psychiatric Association.

The diagnosis of ADHD should be formulated with great care and only if the signs of inattention or hyperactivity take on excessive importance in relation to the age and mental development of the child. In fact, inattention in the school setting may occur when children endowed with high intelligence are placed in a under stimulating environment culturally and psychologically.

ADHD affects approximately 5-10% of school-age children. According to epidemiological studies conducted in the United States and Europe, ADHD is 10 times more frequent in males than in females.

Since learning disorders occur in children who are not capable of fully expressing their feelings, sensations and symptoms, the perception and definition of these disorders is confined to the objective and behavioural signs detected by adults.

Early indications of cognitive and communicative disorders, from the behavioural point of view, may consist in difficulty in controlling impulses, an aimless hyperactive behaviour which gives rise to disciplinary problems, aggressiveness and progressive difficulties at school.

An exhaustive review of learning disorders and attention deficits is provided in the Merck Manual of diagnosis and therapy, third Italian edition (1995), pp. 2213-2219.

The drugs most frequently used for the treatment of ADHD are methylphenidate and clonidine.

Methylphenidate has been the subject of various clinical studies which have demonstrated its efficacy both in hyperactivity and in attention deficit. However, cases of rebound hyperactivity and increased impulsivity have often been reported.

Other adverse or side effects are dilation of the pupils, headache, decreased appetite, difficulty in falling asleep, nocturnal enuresis, drowsiness and reduced systolic and diastolic pressure.

The adverse effects of clonidine include particularly drowsiness, interruption of nocturnal sleep often accompanied by nightmares, nausea, decreased appetite and reduction of diastolic and systolic pressure which occurs more frequently than in the case of treatment with methylphenidate.

The use of L-carnitine has recently been proposed with favourable results [see US patent 5,869,528 (Sigma-Tau)].

The object of present the invention is to provide a composition for the prevention or treatment of ADHD which does not present the unwanted and side effects of methylphenidate and clonidine and which is endowed with greater efficacy than L-carnitine. Since the composition can be used for the prevention or treatment of ADHD, it can take the form and exert the activity of a health food or of an actual medicine, depending upon the supportive or preventive action or the more strictly therapeutic action which the composition exerts according to the particular subjects for whom it is to be used.

The efficacy of the composition is related to the surprising synergistic effect exerted by the combination of its components on the phenomena that underlie the above-described symptoms, such efficacy proving markedly greater than that achievable with the use of its individual components separately.

This object is achieved with the composition according to the present invention comprising as its characterising components:
(a) L-carnitine inner salt or one of its pharmacologically acceptable salts;
(b) acetyl L-carnitine inner salt or one of its pharmacologically acceptable salts; and
(c) a huperzine.

The huperzine is preferably selected from the group comprising huperzine A and huperzine B or mixtures thereof. An extract of *Huperzia serrata* can be used as a mixture of said huperzines.

The activity exerted by the alkaloids of *Huperzia serrata* (*Thunb.*) [or *Lycopodium serratum* (*Thunb.*)] at central nervous system level have been known for some time.

The use of this plant originally coming from China was already known in popular medicine for the treatment of contusions and muscle pains and for the treatment of schizophrenia, but the description of the structures of the two main alkaloids present in it, i.e. huperzine A and huperzine B, is relatively recent (Lin J.S., Can. J. Chem., 64:837, 1986). Immediately after this, Wang (Wang Y.E., Acta Pharmacol. Sinica, 7:110, 1986) described their potent anticholinesterase action which paved the way for the pharmacological study of these alkaloids.

The potent, selective anticholinesterase activity of huperzine A and huperzine B immediately prompted research into these alkaloids in the field of substances active against Alzheimer's disease.

Recently, research into the pathogenesis of this disease has, in fact, focused, above all, on the study of anticholinesterases endowed with selective activity and low toxicity, since it has been found that it is mainly the cerebral cholinergic structures that present the greatest degree of both organic and functional deterioration in ageing processes and in Alzheimer's disease.

Substances capable of blocking central cholinergic activity are, in fact, capable of producing a picture similar to that of senile dementia. Cholinergic antagonists and acetylcholinesterase inhibitors, on the other hand, are capable of improving the geriatric symptoms relating to memory.

Comparative studies conducted with huperzine A have demonstrated its greater efficacy and superior tolerability compared to drugs such as tacrine. Greater efficacy has also recently been observed for huperzine B. In addition to anticholinesterase activity, other studies have demonstrated the ability, for example, of huperzine A to improve memory in experimental animals as well as in patients with Alzheimer's disease.

It has also been observed that superstimulation of glutamate receptors, regarded as one of the components that contribute towards the degenerative lesions encountered in Alzheimer's or Parkinson's disease, may be inhibited by the administration of huperzine A. It has also been observed that huperzine A, in addition to its effect on acetylcholinesterases, is also capable of acting as an antagonist on NMDA (N-methyl-D-aspartate) receptors at the level of the cerebral cortex, which also contribute to the cerebral neurodegenerative picture.

The pharmacological and the clinical effects appear comparable with both natural and synthetic huperzine A.

The use of huperzine, alone or in combination with other active ingredients, had never previously been proposed for the prevention/treatment of ADHD.

It has now surprisingly been found that a composition comprising as its characterising components a combination of:
(a) L-carnitine inner salt or one of its pharmacologically acceptable salts;
(b) acetyl L-carnitine inner salt or one of its pharmacologically acceptable salts; and
(c) a huperzine, preferably selected fromn the group consisting of huperzine A, huperzine B and mixtures thereof,
is extremely effective in the prevention and/or treatment of learning disorders in children suffering from Attention Deficit/Hyperactive Disorder (ADHD) owing to the unexpected potent synergistic effect exerted by its components.

Preferably, the weight-to-weight ratio of components (a) + (b) to component (c) ranges from 1:10⁻² to 1:10⁻⁶.

The composition which is the subject matter of the present invention can be used either as a health food or dietary supplement with a mainly preventive action or as a medicine for the treatment of frank pathological states.

The surprising synergistic effect which is achieved with the combination of L-carnitine, acetyl L-carnitine and the aforesaid huperzines has been demonstrated by several pharmacological tests (some of which are described here below) chosen in such a way as to be strongly predictive for the practical use of this composition in both the preventive/nutritional field and in the more strictly therapeutic field.

### Anticholinesterase activity tests

The activity of the composition according to the invention was evaluated by assaying its ability to inhibit cerebral acetylcholinesterase in mice pretreated orally for eight consecutive days with 200 mg/kg of L-carnitine and 50 mg/kg of acetyl L-carnitine as compared to animals treated with huperzine A alone. After eight days, the animals pre-treated with L-carnitine and acetyl L-carnitine were administered huperzine A at two different doses by the gastric route. The animals were then sacrificed, and the frontal cortex and left hemisphere of the brain were homogenised cold in buffer solution. Prior to the test, the homogenate was incubated with butyrylcholinesterase inhibitors. The anticholinesterase activity was measured according to the spectrophotometric method described by Ellman (Ellman G.L., Biochem. Pharmacol., 7:88, 1961). Acetylcholinesterase 0.3 mmol/L was used as substrate and the mixture of enzyme, substrate and sodium phosphate buffer was incubated in a total volume of 4 mL at 37°C for 8 min. The reaction was blocked with the addition of 1 mL of 3% sodium dodecylsulphate and then with the further addition of 1 mL of 0.2% dithionitrobenzoic acid (DTNB). Spectrophotometric measurements were then taken at 400 nm. From the results reported in Table 1 it appears that pretreatment with L-carnitine and acetyl L-carnitine brought about a more pronounced inhibitory effect of huperzine A on cerebral acetylcholinesterase, preferentially at the level of the cortex rather than at the level of the cerebral mass.

Pretreatment with L-carnitine plus acetyl L-carnitine was therefore capable of increasing the activity of huperzine A.

**Table 1**

| Anticholinesterase activity at cerebral level | | |
|---|---|---|
| Treatment nmol/kg | Percentage inhibition of acetylcholinesterase | |
| | Cerebral | Cortex |
| huperzine A | | |
| 60 | 22±2.5 | 39±5.1 |
| 40 | 15±1.1 | 29±2.6 |

| huperzine A (L-carnitine + acetyl L-carnitine) | | |
|---|---|---|
| 60 | 24±3.4 | 44±4.4 |
| 40 | 18±2.1 | 39±2.9 |

### Effect on glutamate-induced cortical neuronal toxicity

As is known, persistent stimulation of glutamate receptors induces a neurotoxic effect leading to the death of neuronal cells: this is one of the mechanisms regarded as being responsible for the pathogenesis of Alzheimer's disease. The aim of these tests was to observe whether preincubation of cortical neuronal cells with L-carnitine + acetyl L-carnitine alone or in combination with huperzine A may lead to a reduction of glutamate-induced toxicity and to greater survival of neuronal cells.

In these tests, cortical neurons isolated from mouse foetuses were used, according to the method described by Lin [Lin L., J. Nanjing Univ. (Natural Sciences Edition) 31:514, 1995 - Lin L., Acta Pharmacol. Sinica, 17:221, 1996]. After isolating the cells, they were suspended on plates with a density of 6.4 × 10⁸ cells/m² surface. To prevent proliferation of non-neuronal cells, arabinoside (10 nmol·L⁻¹) was added to the cytosine culture. To determine the glutamate toxicity, the cells were incubated in a modified Locke solution at 37°C for 30 minutes with monosodium glutamate at concentrations ranging from 0.5 to 1.5 nmol·L⁻¹.

The solutions of L-carnitine/acetyl L-carnitine or huperzine A were added to the Locke solution half an hour before monosodium glutamate. Death of the incubated cells was determined by staining with MTT (3,4,5-dimethylthiazolyl-2,5-tetrazolium bromide) and their viability by the release of lactate dehydrogenase (LDH) according to the procedure disclosed by Green (Green L. M., J. Immunol. Methods, 70:257, 1984).

The results of these tests indicate that, whereas the neuronal cells which remained in contact with monosodium glutamate alone (1 nmol·L⁻¹) present a survival rate of 20%, both the cells placed in contact with glutamate and L-carnitine/acetyl L-carnitine (5 nmol·L⁻¹) and those placed in contact with huperzine A (10 nmol·L⁻¹) show a 30% survival rate; whenever L-carnitine/acetyl L-carnitine are incubated together with huperzine A prior to glutamate, the cell survival is above 70%, thus demonstrating a potent synergistic effect of L-carnitine/acetyl L-carnitine and huperzine A in protecting neuronal cells against glutamate toxicity.

### Glutamate excitability effect

Excitatory amino acids such as glutamate, if administered by intracerebral injection, produce a series of behavioural effects, the most evident of which is hypermotility.

To evaluate the effect of huperzine A and L-carnitine/acetyl L-carnitine and of a combination of these, mice received intracerebral injections of increasing doses of glutamate according to the method described by Lama (Lama E., Acta Pharmacol. Sinica, 9:252, 1988). The increase in spontaneous motility of these animals placed in a box with transparent walls was then evaluated by means of a photoelectric cell which recorded the number of passes for a period of five minutes. The motility test was conducted both in control animals and in animals treated one hour prior to injection of glutamate with huperzine A and with L-carnitine/acetyl L-carnitine amd with a combination of these compounds. The results of these tests (see Table 2) indicate that treatment with huperzine A reduces the increase in motility induced by glutamate in mice, but that the greatest reduction is that obtained by administration of the combination of huperzine A and L-carnitine/acetyl L-carnitine.

**Table 2**

| Glutamate excitability tests | | |
|---|---|---|
| Glutamate ng/mouse | Treatment | N. passes in 5 min observation |
| 0 | --- | 127±22 |
| 1 | --- | 241±35 |
| 5 | --- | 364±39 |
| 1 | huperzine A, 0.05 mg/kg | 226±24 |
| 5 | huperzine A, 0.05 mglkg | 296±16 |
| 1 | L-carnitine/acetyl L-carnitine^{(*)} | 231±26 |
| 5 | L-carnitinelacetyl L-carnitine^{(*)} | 305±33 |
| 1 | huperzine A. 0.05 mg/kg + L-carnitine/acetyl L-carnitine^{(*)} | 196±16 |
| 5 | huperzine A, 0.05 mg/kg + L-carnitinelacetyl L-carnitine^{(*)} | 216±22 |

| | | |
|---|---|---|
| (*) 200 mg/kg of L-carnitine + 50 mg/kg of acetyl L-carnitine | | |

### Clinical Study

30 children diagnosed with ADHD were enrolled in the study twenty were males and ten females, all of them living in a family home.

The diagnostic selection criteria according to DSM-IV were established by paediatric psychiatrists or paediatric psychologists in a center specialized for the treatment of ADHD.

Ten children out of 30 showed Attention Deficit signs, nine hyperactive/impulsivity signs and the others the two combined disorders. The children were not administered stimulant medicaments. All standard treatments were stopped and replaced by the daily administration of 60 mg L-carnitine, 15 mg acetyl L-carnitine and 4 µg huperzine per kg of body weight.

The clinical evaluation was done by means of Groningen's Parent Observation Scale [see Boorsma S. (1990). The parent version of the Groningen Behaviour Observation scale: Factor structure and norms. In A. F. Kalverboer (Ed.) Developmental Biopsycology: Experimental and observational studies in children at risk (pp. 293-298). Ann. Arbor: University of Michigan Press] and two Teacher Observation Scales, namely Groningen's scale [see Vaessen W. (1990). The teacher version of Groenings Behaviour Observation scale: Factor structure and norms. In A. F. Kalverboer (Ed.) Developmental Biopsycology: Experimental and observational studies in children at risk (pp. 287-291). Ann. Arbor: University of Michigan Press] and Conner's scale [see Werry J.S. Sprague R.L. & Cohen M. N. (1975), Conners' Teacher Rating Scale for use in drug studies with children: An empirical study. Journal of Abnormal Child Psychology 3. 217-299].

The response to the treatment with the composition of the invention was evaluated as a global clinical impression coming out from the three aforesaid rating scales.

The response was rated positive only when the target signs (15 and 15 for the Groninger Behaviour Observation scale, parent version and teacher version, respectively, and 39 for Conner's Teacher Rating Scale) disappeared or decreased markedly.

Based on these evaluation criteria, at week twelve (end of tretment) the response was considered positive in nine children prevailingly affected by Attention Disorders, eight children prevailingly affected by Hyperactivity/Impulsivity Disorders and ten children affected by the combined symptoms. The positive response accounts for a total of twenty-seven patients out of thirty i.e. 90% of the treated population. This result is markedly superior to that of the clinical study carried out with L-carnitine alone reported in the aforesaid US patent 5,869,528.

Some non-limiting examples of compositions according to the present invention are given hereinbelow.

| | | | |
|---|---|---|---|
| 1) | L-carnitine | mg | 300 |
| | Acetyl L-carnitine | mg | 70 |
| | Huperzine A | pg | 30 |
| | | | |
| 2) | L-carnitine | mg | 300 |
| | Acetyl L-carnitine | mg | 70 |
| | Huperzine A | µg | 15 |
| | Huperzine B | µg | 15 |
| | | | |
| 3) | L-carnitine | mg | 300 |
| | Acetyl L-carnitine | mg | 70 |
| | Extract from *Huperzia serrata* titred in huperzine A equal to | µg | 30 |
| | | | |
| 4) | L-carnitine | mg | 300 |
| | Acetyl L-carnitine | mg | 70 |
| | Huperzine A | µg | 30 |
| | Vit. E | mg | 10 |
| | Coenzyme Q₁₀ | mg | 30 |
| | | | |
| 5) | L-carnitine | mg | 350 |
| | Acetyl L-camitine | mg | 70 |
| | Huperzine A | µg | 25 |
| | Coenzyme Q₁₀ | mg | 25 |
| | β-carotene | mg | 10 |
| | Vit. E | mg | 10 |
| | | | |
| 6) | L-carnitine | mg | 300 |
| | Acetyl L-carnitine | mg | 70 |
| | Huperzine A | µg | 15 |
| | Huperzina B | µg | 15 |
| | Phosphorylcholine | mg | 100 |
| | Glicerytphosphorylcholine | mg | 100 |
| | Phosphorylserine | mg | 100 |
| | Vit. E | mg | 10 |
| | Coenzyme Q₁₀ | mg | 25 |
| | | | |
| 7) | L-carnitine | mg | 250 |
| | Acetyl L-camitine | mg | 60 |
| | Huperzine A | µg | 20 |
| | Huperzine B | µg | 20 |
| | Tryptophan | mg | 100 |
| | Serine | mg | 50 |
| | Tyrosine | mg | 100 |
| | Glutamine | mg | 50 |
| | Vit. E | mg | 10 |
| | β-carotene | mg | 10 |
| | Coenzyme Q₁₀ | mg | 25 |
| | Selenomethionine | µg | 50 |
| | Magnesium | mg | 10 |

What is meant by a pharmacologically acceptable salt of the various aforesaid carnitines mentioned in the present specification is, in addition to the respective "inner salts", any salt of these with an acid which does not give rise to unwanted toxic or side effects. These acids are well known to pharmacologists and to experts in pharmaceutical technology.

Non-limiting examples of such salts are the following: chloride; bromide; iodide; aspartate, acid aspartate; citrate, acid citrate; tartrate; phosphate, acid phosphate; fumarate; acid fumarate; galactarate; glycerophosphate; glucose phosphate; lactate; maleate; acid maleate; orotate; oxalate, acid oxalate; sulphate, acid sulphate; trichloroacetate; trifluoroacetate and methane sulphonate.

Among these salts, L-carnitine cid fumarate (US 4,602,039) and acetyl L-carnitine galactarate (US 5,952,379) are particularly preferred.

A list of FDA-approved pharmacologically acceptable acids is given in Int. J. Pharm., 33, 1986, 201-217, the latter publication being incorporated in the present specification by reference.

The composition of the invention may further comprise vitamins, coenzymes, mineral substances, aminoacids, antioxidants and proteins. The composition may be manufactured in the form of tablets, lozenges, capsules, pills, granulates, syrups, herb teas, vials or drops.

## Claims

1. A combination composition comprising:
(a) L-carnitine inner salt or a pharmacologically acceptable salt thereof; and
(b) acetyl L-carnitine inner salt or a pharmacologically acceptable salt thereof; and
(c) a huperzine.

2. The composition of claim 1 wherein huperzine is selected from the group consisting of huperzine A, huperzine B and mixtures thereof.

3. The composition of claims 1 and 2 wherein the huperzine is in the form of an extract of *Huperzia serrata*.

4. The composition of claims 1-3, wherein the weight ratio (a)+ (b):(c) ranges from 1:10⁻² to 1:10⁻⁶.

5. The composition of anyone of the preceding claims, wherein the pharmacologically acceptable salt of L-carnitine or acetyl L-carnitine is selected from the group comprising: chloride; bromide; iodide; aspartate, acid aspartate; citrate, acid citrate; tartrate; phosphate, acid phosphate; fumarate; acid fumarate; galactarate; glycerophosphate; glucose phosphate; lactate; maleate; acid maleate; orotate; oxalate; acid oxalate; sulphate, acid sulphate; trichloroacetate; trifluoroacetate and methane sulphonate.

6. The composition of anyone of the preceding claims, further comprising vitamins, sugars, coenzymes, mineral substances, aminoacids, peptides, antioxidants and proteins.

7. The composition of claim 6, wherein the coenzyme/antioxidant is selected from the group comprising coenzyme Q₁₀, Vitamin E, selenium, β-carotene or mixture thereof.

8. The composition of anyone of the preceding claims, orally or parenterally administrable, in the form of dietary supplement.

9. The composition of anyone of the preceding claims, orally or parenterally administrable, in the form of a medicament.

10. Use of a combination preparation comprising:
(a) L-carnitine inner salt or a pharmacologically acceptable salt thereof; and
(b) acetyl L-carnitine inner salt or a pharmacologically acceptable salt thereof; and
(c) a huperzine selected from the group consisting of huperzine A, huperzine B or mixture thereof or an extract of *Huperzia serrata,*
for preparing a dietary supplement or medicament for the prevention/treatment of learning disorders in children suffering from Attention Deficit/Hyperactive Disorder (ADHD).

11. The use according to claim 10, wherein component (a), (b) and (c) are administered orally or parenterally.

12. The use according to claim 11, wherein the total daily administered amount per kg of body weight of component (a) is from about 20 to about 80 mg; of component (b) is from about 5 to about 30 mg and of component (c) is from about 2 to about 4 µg, respectively.

## Patentansprüche

1. Kombinationszusammensetzung, umfassend:
(a) inneres Salz von L-Carnitin oder ein pharmakologisch annehmbares Salz davon;
(b) inneres Salz von Acetyl-L-carnitin oder ein pharmakologisch annehmbares Salz davon und
(c) ein Huperzin.

2. Zusammensetzung nach Anspruch 1, wobei Huperzin aus der Gruppe, bestehend aus Huperzin A, Huperzin B und Gemischen davon, ausgewählt ist.

3. Zusammensetzung nach Anspruch 1 und Anspruch 2, wobei das Huperzin in Form eines Extraktes von Huperzia serrata vorliegt.

4. Zusammensetzung nach den Ansprüchen 1 bis 3, wobei das Gewichtsverhältnis (a)+(b):(c) im Bereich von 1:10⁻² bis 1:10⁻⁶ liegt.

5. Zusammensetzung nach einem der vorangehenden Ansprüche, wobei das pharmakologisch annehmbare Salz von L-Carnitin oder Acetyl-L-carnitin aus der Gruppe, umfassend Chlorid; Bromid; Iodid; Aspartat, saures Aspartat; Citrat, saures Citrat; Tartrat; Phosphat, saures Phosphat; Fumarat, saures Fumarat; Galactarat; Glycerophosphat; Glucosephosphat; Lactat; Maleat, saures Maleat; Orotat; Oxalat, saures Oxalat; Sulfat, saures Sulfat; Trichloracetat; Trifluoracetat und Methansulfonat, ausgewählt ist.

6. Zusammensetzung nach einem der vorangehenden Ansprüche, die außerdem Vitamine, Zucker, Coenzyme, Mineralstoffe, Aminosäuren, Peptide, Antioxidanzien und Proteine umfaßt.

7. Zusammensetzung nach Anspruch 6, wobei das Coenzym/Antioxidans aus der Gruppe, umfassend Coenzym Q₁₀, Vitamin E. Selen, β-Carotin oder Gemische davon, ausgewählt ist.

8. Zusammensetzung nach einem der vorangehenden Ansprüche, die in Form einer Nahrungsergänzung oral oder parenteral verabreichbar ist.

9. Zusammensetzung nach einem der vorangehenden Ansprüche, die in Form eines Medikaments oral oder parenteral verabreichbar ist.

10. Verwendung eines Kombinationspräparats, umfassend:
(a) inneres Salz von L-Carnitin oder ein pharmakologisch annehmbares Salz davon;
(b) inneres Salz von Acetyl-L-carnitin oder ein pharmakologisch annehmbares Salz davon und
(c) ein Huperzin, ausgewählt aus der Gruppe bestehend aus Huperzin A, Huperzin B oder einem Gemisch davon oder einem Extrakt von Huperzia serrata,
zur Herstellung einer Nahrungsergänzung oder eines Medikaments zur Prävention/Behandlung von Lernstörungen bei Kindern, die an Aufmerksamkeitsdefizit-Hyperaktivitäts-Störung (Attention Deficit/Hyperactive Disorder (ADHD)) leiden.

11. Verwendung nach Anspruch 10, wobei die Komponente (a), (b) und (c) oral oder parenteral verabreicht werden.

12. Verwendung nach Anspruch 11, wobei die täglich verabreichte Gesamtmenge pro kg Körpergewicht an Komponente (a) etwa 20 bis etwa 80 mg ist; an Komponente (b) von etwa 5 bis etwa 30 mg ist und an. Komponente (c) etwa 2 bis etwa 4 µg ist.

## Revendications

1. Composition de combinaison comprenant :
(a) un sel interne de L-carnitine ou un sel pharmacologiquement acceptable de celle-ci ;
(b) un sel interne d'acétyl-L-carnitine ou un sel pharmacologiquement acceptable de celle-ci ; et
(c) une huperzine.

2. Composition selon la revendication 1, dans laquelle l'huperzine est choisie dans le groupe constitué de l'huperzine A, de l'huperzine B et de leurs mélanges.

3. Composition selon les revendications 1 et 2, dans laquelle l'huperzine est sous la forme d'un extrait de *Huperzia serrata.*

4. Composition selon les revendications 1 à 3, dans laquelle le rapport en masse (a)+(b):(c) varie de 1:10⁻² à 1:10⁻⁶.

5. Composition s elon l'une q uelconque d es r evendications p récédentes, d ans laquelle le sel pharmacologiquement acceptable de L-camitine ou d'acétyl-L-carnitine est choisi parmi le groupe comprenant : le chlorure ; le bromure ; l'iodure ; l'aspartate, l'aspartate acide ; le citrate, le citrate acide ; le tartrate ; le phosphate, le phosphate acide ; le fumarate, le fumarate acide ; le galactarate ; le glycérophosphate ; le phosphate de glucose ; le lactate ; le maléate, le maléate acide ; l'orotate ; l'oxalate ; l'oxalate acide ; le sulfate, le sulfate acide ; le trichloroacétate ; le trifluoroacétate et le sulfonate de méthane.

6. Composition selon l'une quelconque des revendications précédentes, qui comprend en outre des vitamines, des sucres, des coenzymes, des substances minérales, des acides aminés, des peptides, des antioxydants et des protéines.

7. Composition selon la revendication 6, dans laquelle la coenzyme ou l'antioxydant est choisi dans le groupe comprenant la coenzyme Q₁₀, la vitamine E, le sélénium, le β-carotène ou leurs mélanges.

8. Composition selon l'une quelconque des revendications précédentes, administrable par voie orale ou parentérale, sous la forme d'un complément alimentaire.

9. Composition selon l'une quelconque des revendications précédentes, administrable par voie orale ou parentérale, sous la forme d'un médicament.

10. Utilisation d'une composition de combinaison comprenant :
(d) un sel interne de L-camitine ou un sel pharmacologiquement acceptable de celle-ci ;
(e) un sel interne d'acétyl-L-carnitine ou un sel pharmacologiquement acceptable de celle-ci ; et
(f) une huperzine choisie dans le groupe constitué de l'huperzine A, de l'huperzine B et de leurs mélanges, ou un extrait de *Huperzia serrata,*
pour la préparation d'un complément alimentaire ou d'un médicament destiné à la prévention ou au traitement de troubles de l'apprentissage chez des enfants souffrant d'hyperactivité avec déficit de l'attention (ADHD).

11. Utilisation selon la revendication 10, dans laquelle les composés (a), (b) et (c) sont administrés par voie orale ou parentérale.

12. Utilisation selon la revendication 11, dans laquelle la quantité journalière totale administrée par kg de poids corporel du composé (a) est d'environ 20 à environ 80 mg, celle du composé (b) est d'environ 5 à environ 30 mg et celle du composé (c) est d'environ 2 à environ 4 µg.
